# EUROPEAN PATENT APPLICATION

(11) **EP 3 372 521 A1**
(43) Date of publication of application: **12.09.2018**
(21) Application number: 16861647.2
(22) Date of filing: 07.10.2016
(51) Int. Cl.: B65D 47/04, A61K 35/12, A61K 8/97, A61J 1/20, B65D 47/26

(54) **FLEXIBLE DISPENSING CONTAINER FOR APPLYING HAIR FIBRE OR CONCEALER**

(30) Priority: 08.11.2015 ES 201531224 U
(71) Applicant: Torrano Pageo, Maria Irene, 30100 Murcia (ES)
(72) Inventor: VIDAL CANO, Francisco Javier, 30100 Murcia (ES); TORRANO PAGEO, Maria Irene, 30100 Murcia (ES)
(86) International application number: PCT/ES2016/070717
(87) International publication number: WO 2017/077148

(57) **Abstract**

The invention relates to a flexible dispensing container for applying hair fibre or concealer, not suitable for other powder products, and belongs to the hairdressing industry. The invention comprises: a flexible part, called a flexible dispensing container (1), which houses the hair fibre. Said part is joined to an immovable, fixed, quantity-regulating filter (2) into the upper part of which is inserted an upper, movable, quantity-regulating filter (3) that rotates on the immovable filter. The filters have a diameter of approximately 1.5 cm and are cylindrical. By means of both filters, the fibre is dispensed according to quantity levels, according to the hair loss. There are three quantity levels: level 1 (7), level 2 (8) and level 3 (9). The filters comprise 8 and 12 holes (4, 5) which, when the quantity level is chosen, produce a combination of holes, the position and opening thereof causing the hair fibre to be applied to each of the individual's hairs, in the desired area.

## Description

### SPECIFIC TECHNICAL FIELD

This invention belongs to the field of cosmetics, and more specifically to that of hairdressing; it hides a lack of hair through the application of hair fiber in areas of the hair where such lack is present.

This invention is called flexible dispenser for the application of hair fiber or hair concealer, and is composed of several parts; in its lower area, by a flexible part called flexible dispenser, which is where the hair fiber is stored.

This flexible part is joined at approximately the same height as the central part of the whole container, object of the invention, to a fixed quantity regulating filter. At the same time, it has an upper quantity regulating filter inserted in its upper part; through both filters it is possible to dispense and choose different amounts of hair fiber or hair concealer to apply, depending on individual needs.

There are several holes in each of the filters which, upon selecting the different application levels, create a combination of holes whose position and aperture have been studied according to the amount of fiber which one wishes to apply and the composition characteristics and nature of the hair fiber for its correct application.

There are three quantity levels of hair fiber to select, level 1, level 2, and level 3; these are configured to precisely, exactly, and cleanly dispense the quantity of hair fiber applied to each of the individual's hairs; this hair-fiber dispensing system with quantity levels causes the hair fiber to pass through a combination of holes located in the fixed quantity regulating filter and in the upper quantity regulating filter, resulting in an application which is without lumps, not heavy, very fine, and which does not deposit the hair in undesired places or areas of the head (forehead, face, etc.), thereby achieving the desired result of the lack of hair being unnoticeable to others.

### INVENTION ANTECEDENTS

Currently, due to stress, eating habits, and other causes hair loss is a widespread problem, both for men and women, which gives the appearance of premature aging. There are many methods for avoiding, reducing, and concealing hair loss; these range from pharmaceutical products to implants, but the high cost of the alternatives make concealing baldness of the most economical ways to help keep one looking young.This is why applying hair fiber, with an impeccable and imperceptible result, would alleviate and improve this social problem.

The invention, the flexible dispenser for applying hair fiber or hair concealer, is meant for and designed to contain and apply only hair fiber according to its nature, composition, and the application characteristics of the same (The hair fiber should be applied without it being too heavy, without lumps, very very finely and should be imperceptible once applied to each of the individual's hairs); this invention is not meant for the application of powder makeup, talcum powder or any other powder product, distinct from hair fiber, due to the composition of the different products and the purpose, final result and application of them as well as for that of the hair fiber itself.

Currently, there are numerous containers which contain and dispense talcum powder, makeup and other powder products, as well as other containers which contain or are used to apply hair fiber. Given the distinct composition of said products and therefore the nature and conservation behavior, their composition, and the application method of each of them via the containers holding them, the flexible dispenser for applying hair fiber or hair concealer, object of invention, has neither the utility nor the characterization of any existing ones on the market due to its inventive and novel nature

Each product has a purpose, and is used according to the needs which one intends to satisfy with its application; none of the aforementioned products are applied in the same manner, nor for the same end, nor is it necessary or efficacious to apply the same amount, nor with the same application precision. Likewise, with the passage of time, not all of the products keep the same conservation properties for their correct use at a later date; for this reason they cannot all be conserved in the same manner, nor applied with the same technique to obtain the desired result.

According to the invention antecedents, there are currently numerous dispensing devices which for the most part make us of the flexibility of part of the container to propel the powder product which they contain, as well as different mechanisms and aperture filters and closures; nonetheless, just like the flexible dispenser for applying hair fiber or hair concealer, object of this invention, each of them incorporates an inventive end solution to apply the product for which they have been invented.

The containers for other powder products have been tried for the application of hair fiber, as well as all other existing containers containing hair fiber, without achieving the desired result, in any way; the flexible dispenser for applying hair fiber or hair concealer, object of this invention, perfectly applies hair fiber to each of the individual's hairs; it does so precisely and exactly, without it being too heavy, without lumps, and without it being deposited on any other place, area or hair other than that desired by the individual; this results in a result which is therefore perfect, imperceptible, and desired; as a result, nobody knows that you use the product.

Below are the details of numerous containers currently available which are known to use, in one way or another, the flexibility of the container as an air pump, some external device applied to the container, as well as some type of opening and closing system for the same, in order to apply the product or products for which they have been invented.
- Container for powder cosmetics, with document number US2015047670A1**.**
   The techniques utilized prior to this invention involved using fingers to apply for product; it was therefore necessary to create a new, more hygienic container to apply said makeup, with some tool adapted to the container which would allow one to apply it correctly. As such, this patent includes in its invention a sponge for said application, located at the end of the upper part of the container. The patent is characterized by the lower part having an angular slot in which one introduces makeup in soft-disk form; the makeup is later deposited on a sponge located on the upper rotation member thanks to rotation members which open and close the container, without measuring the amount of makeup to be applied; the sponge is then used to apply the powder cosmetic to the skin.

Our object of invention, the flexible dispenser for applying hair fiber or hair concealer, is nothing like this invention given that in the container with document number US2015047670A1 the angular slot deposits the makeup on a disk; likewise, the available rotation members expel the makeup by opening and closing the container, with the makeup being deposited onto the application sponge located in the upper part of the container.
The flexible dispenser for applying hair fiber or hair concealer, object of our invention, does not deposit hair fiber onto any application tool, as is the case with the previously described patent; this is because it would be of no use in the case of hair fiber; using any tool like a sponge would result in hair fiber coming out in a manner which would be impossible to apply to each individual hair.

With the flexible dispenser for applying hair fiber or hair concealer, object of invention, the individual selects that amount they wish to apply to the area of the head where they wish to apply the hair fiber; there are three different quantity levels: level 1, level 2, and level 3. Afterwards, the container is positioned upside down, and the hair fiber is deposited on the upper part of the fixed quantity regulating filter; then, by pressing lightly on the lower part, the flexible dispenser sends the hair fiber to the openings which the holes in the fixed quantity regulating filter and upper quantity regulating filter have left open to be combined according to the amount of hair fiber to apply. This way, the fiber is pulverized by passing through the resulting openings or holes. The hair fiber is thinned, which prevents the fiber from coming out lumpy. Due to the studied measurements of the diameters of the filters, their position in the filters, and the combination of the apertures resulting from the upper quantity regulating filter being turned on the fixed quantity regulating filter, according to amount selected to be applied to the specific area to be treated, the hair fiber comes out is deposited exactly on the area desired by the individual, and in no others, and on each one of the hairs where hair loss needs to be concealed; this means a perfect result which meets the user's expectations regarding the product, which is none other than to conceal baldness and for other people not to notice that it has been applied. Using the container, object of invention, for the application of any other product apart from hair fiber will not produce the desired result.
- Another existing precursor is the device for dispensing powder such as talcum powder, with document number WO2004105961A1, in the description of said device reference is made to another flexible container used to dispense talcum powder, as is the case with US patent US4730751, compressible powder dispenser bottle; this invention has flexible walls and a foam pad for the smooth and uniform dispensing of powder is different from the aforementioned American patent because it uses a distribution nozzle for talcum powder with openings and an intermediate reservoir which holds the talcum powder until its next application; every time, as with the patented American container, the walls of the invented container are pressed in order to expel the talcum powder, located in the intermediate reservoir, with successive squeezes of the container's flexible walls. None of these containers allow one to choose the amount to apply, nor do they have the combination of several holes in the fixed quantity regulating filter and the upper quantity regulating filter which, according the amount of hair fiber one wishes to apply, combine in order to create the openings for the hair fiber to be expelled from in amount needed, according to the area to treat; they also do not have the product passing through a determined number of openings or holes according the level of hair fiber selected so that it comes out without being too heavy, without lumps, and in powder form for an imperceptible and invisible application. Furthermore, both patented containers are meant to be used with talcum powder and not hair fiber.

Just as is the case with the powder-makeup container mentioned in the previous point, for the case of the precursors for the state of art, our object of invention, flexible dispenser for the application of hair fiber or hair concealer, has nothing in common with the container with document number WO2004105961A1 **nor with the container with document number** US4730751A cited in its records, given that the container object of invention, in addition to not being meant for applying talcum powder, since it has a composition, conservation, and application purpose which are very different to hair fiber, our flexible dispenser for the application of hair fiber or hair concealer, object of this invention, has no intermediate reservoir or pad with which to deposit or filter the hair fiber prior to it being expelled since this would be absolutely useless due to the fact that doing so would cause the hair fiber to become thick and lumpy upon being applied.

In order for hair fiber to be correctly and precisely applied, so that other individuals do not notice it, it must be deposited very finely on each hair in powder form; the amount which one wishes to apply to the exact area of the head where one wishes to place the hair fiber must be selected, with the different quantity levels being level 1, level 2, and level 3 so that it remains invisible. Afterwards one must position the container upside down and lightly squeeze it in order to send the hair fiber to the fixed quantity regulating filter and the upper quantity regulating filter holes. This way, the hair fiber is again pulverized and, upon coming into contact with the resulting combination of holes, according to the position and opening of the holes based on the amount selected, is thinned and applied without clumps. One must keep in mind the approximate diameter of the filters, 1.5 centimeters, the holes, the position and opening of the same, and the combination of the same according to the amount to be applied for the specific area to be treated in order to obtain the desired result, which expels and deposits on the desired area, and not on any other, and on each of the hairs where one needs to conceal hair loss; this achieves the perfect and hoped-for result of the product, which is nothing less than concealing baldness without others noticing that the hair fiber has been applied, as previously described.
- Another existing patent is the entrainment powder container with document number US4261488A which makes reference to a compression container for entraining powder for the application of a powdery product, which when squeezed, has a bypass tube which opens up into a spherical hollow chamber which acts as a mixing chamber where the powdery product is deposited; it also has a covering which is opened to remove lumps by opening and closing. At the same time, we could mention the container called dispenser container apt for children and adults, with document number GB2328926A this is a dispenser made of flexible material designed to apply any type of product which one can squeeze with one's hand and place any type of closure, its documentation cites several existing ones; nonetheless, it does not mention a level-quantity regulator. It also mentions that it can be placed in a vertical position with the closure up or down and emphasizes the fact that the container size can be grasped by children and adults.

In short, the flexible dispenser for the application of hair fiber or hair concealer, object of this invention, is totally different from those mentioned in the previous paragraphs; it does not have a mixing chamber; it does not base its application on the flexibility of the container, as is the case with the invention antecedents previously commented upon; this is due to the already mentioned fact that, due to the compositional nature of hair fiber, that if the same were deposited in any device or channel, it would thicken and become lumpy and would become blocked upon passing through any channel, system, or bypass tube.

Furthermore, the innovation of this invention, the flexible dispenser for the application of hair fiber or hair concealer, does not depend on the position, nor the pressure on the flexible container to expel the product. Instead what causes the dispersion and pulverize been the dispersion and pulverization necessary for the hair fiber to be correctly applied is caused by placing the container upside down without shaking it; this makes the fiber reach the upper part of the fixed quantity regulating filter and the upper quantity regulating filter; it then passes through the holes which leave a plurality of the holes located in said filters, which are 1.5 centimeters in diameter, is also by the size of diameters of the filters and holes and the combination of the position and opening of the holes upon selecting the amount to apply, depending on the area to treat, which causes the hair fiber to come out in an optimum fashion for its correct and precise application; this way it is not noticeable and, with the selection of either level 1, level 2, or level 3, remains invisible to others because it is correct y precise application. It is deposited only where the individual wishes it to be so, and on each of the hairs which require the concealment of hair loss; this achieves the desired result for which hair fiber is intended, the concealment of baldness.
- Another existing container is the utility model with document number US2015201732A1 called electric fiber dispenser; it is an electric sprayer which works thanks to a battery-powered motor in the inside of the detachable hair-fiber container and through its automated mechanism and several nozzles which expel the hair fiber via said tubes or conduits. The expulsion speed of the hair fiber varies and the amount of hair fiber is selected by changing two differently sized nozzles.

The description of this utility model mentions several patented hair-fiber sprayers, currently known, which are not efficient and which are different from the object of invention with regards to the application of hair fiber since all of them use specific containers and devices which, via pressure or the application of gas, pump the air which sprays the hair fiber so that it is applied via nozzles, tubes and/or injection jets which expel the hair fiber from the container, just as with the electric dispenser mentioned in this paragraph, as is the case with the patent with document number US6168781 (B1), device for the spraying of hair-fiber, which has a container which stores the hair fiber and a second, detachable tank, attached to the first container with gas-propelled injection mechanism on the first container which discharges the hair fiber onto the hair through the opening or hole of an injection jet; this mechanism requires a separate compressed-air tank, one or two excessively, and uncomfortably, sized hair-fiber containers, and is not practical in terms of application and transportation.
- Likewise, we also mention the container with document number CN202276872U which is defined as a portable corrective device for hairdressing; this is comprised of a rigid container with a filter not adjustable to the quantity levels, which the hair fiber contain, with a nozzle super-imposed on the container which serves as a cover for the same, with the same large size with regards to the diameter of the base of approximately 3 centimeters on which the upper part of the container turns on the upper part of the same; it has something akin to a soft plastic pear or air bag which is bag which is squeezed; this nozzle is connected with the hair-fiber container as an airbag and contains a ventilation grill connected with said container; the airbag unit is provided for by a gas output tube; on the side of the spherical air sacks, the trachea and the air endpoints have a retention-valve spring; this globe structure, ball joint, and windpipe are fixed to the base and push the hair fiber via the conduction of the conduction of the same through the sprayer's conduits or nozzles in order for it to be applied to the hair.

This hair-fiber container currently on the market, can also be used without adding the sprayer, as the already mentioned bulb; this results in the container having a very large diameter, 3 centimeters, a fixed filter which does not adjust to the quantity levels in the exterior of the same diameter as with the container, and with an interval of between 140 and 220 holes.

This must be shaken on the hair to be applied; therefore, the amount of hair fiber or hair concealer applied cannot be regulated, nor can it be applied precisely to the area to be treated; this is because it must be shaken over the area to be treated, unlike the container object of invention which does not require shaking for its application; this means that the aforementioned patented container cannot exactly and precisely deposit hair fiber on each of the individual hairs and therefore cannot achieve the desired final result, which is to conceal hair loss by depositing the hair fiber in such a way so that it is not noticed by others, invisibly and imperceptibly.

Compared with the existing containers previously commented upon and included in the state of the technique, the present invention overcomes the disadvantages of the aforementioned inventions and creates an invention as to the state of the existing technique since the object of this invention, the flexible dispenser for the application of fiber or hair fiber, does not have any nozzle nor any type of conduct, nor an internal channeling mechanism by which hair fiber is expelled; instead, hair fiber is applied by using the container itself since the filters are part of the container itself. It has been confirmed that the very nature of hair fiber makes it thicken and become stuck, forming clumps, when going through different tubes or channeling mechanisms used by other patented systems; this causes, after a short period of time, the nozzles, output channels, or any other mechanism through which the hair fiber is expelled to become obstructed and blocked; this means hair fiber cannot be applied correctly, finely, and as a result cannot be applied to each of the hairs so that it is invisible to the looks of others; as a result, hair loss cannot really be concealed, which is the reason for applying it; it also creates problems, such as having to remove poorly applied product.

Furthermore, using an electric sprayer requires batteries, which bring with them cost and pollution issues; it is also possible that the device will not function correctly when the charge level of the same is below its optimum yield, and as a result will not spray hair fiber finely and precisely; this is a disadvantage which the object of invention, the flexible dispenser for the application of hair fiber or hair concealer, does not have; this is because it is the container itself and not any type of additional device which applies the hair fiber.

Furthermore, he flexible dispenser, object of invention, significantly improves any of the patented hair fiber application devices existing in the market; This is thanks to the ability to select the amount of hair fiber, depending on the level of quantity desired, depending on the area to be treated, without having to change any mouthpiece or behavior, thus eliminating the discomfort and loss of time associated with that action.

The object of invention incorporates, in its own container, the fixed quantity regulating filter and upper quantity regulating filer; there are holes in the fixed quantity regulating filter and the upper quantity regulating filter; their strategic position, the size of the filter diameters of approximately 1.5 centimeters, the position and aperture of the holes and the combination of the same upon the quantity of hair fiber being selected by the individual have been carefully studied and created by our technical team; this way, dosing the hair fiber causes it to pass through a combination of filter holes (between 4 and 8 openings stay open according to quantity level selected); the hair fiber is pulverized and is made fine enough for it to be deposited on the upper part of the fixed quantity regulating filter and on the upper quantity regulating filter.

There are 8 strategically placed holes in the upper part of the fixed quantity regulating filter and 12 holes in the upper quantity regulating filter; combining their position in function of the amount of hair fiber to apply by selecting Level 1, level 2, or level 3 causes the hair fiber to be expelled in accordance with the necessities or lack of each individual through an interval of between 8 and 4 holes which remain open after selecting from the different application levels and with different openings, upon combining the fixed quantity regulating filter holes and the upper quantity regulating filter holes. Both filters have a diameter of approximately 1.5 centimeters in order for the hair fiber to be applied with complete precision on the area to be treated.

The object of invention, the flexible dispenser for the application of hair fiber or hair concealer comes in various sizes, including purse sized.
The object of the present invention allows the user to maximize use and save on the amount of hair fiber utilized to conceal hair loss, applying the same with total precision and control of the amount, flow, direction, and the desired place or area without needing to add sprayers or external channel pieces, nozzles, or intermediate reservoirs to the container. This saves time and space, as well as preventing the hassle of changing nozzles, the cost of changing batteries, blocked nozzles and undesired finishes when applying hair fiber to conceal baldness, with its corresponding removal of hair fiber in order to again apply the same correctly and unnoticeably.
The application of hair fiber via the container object of invention is done with only one hand, without shaking and without losing the precision and exactitude of application, by lightly squeezing the part called flexible dispenser, where the hair fiber is deposited; thanks to its small size, it is easy to apply; the material of the container is very light; it is very easy to use and has an oval and elongated section, not cylindrical; only the filters are cylindrical; these have a diameter of approximately 1.5 centimeters and unlike existing applicators the container object of invention's filters are compact in size.

### EXPLANATION-DISCLOSURE OF THE INVENTION

With the aim of achieving the desired result when apply hair fiber in order to conceal hair loss in a precise and exact manner by depositing the exact amount of needed hair fiber on each of the hairs, and to obtain an invisible result, which conceals hair loss, and to avoid the inconveniences associated with other existing containers or patented models mentioned in the previous paragraphs, the object of this invention proposes a flexible dispenser for the application of hair fiber or hair concealer with the following main advantages:
- The hair fiber is deposited on each hair in the precise and exact space needed, including very small areas of the hair with hair loss, without it being deposited unnecessarily on any other area or place, thereby achieving that it be imperceptible and invisible in order to perfectly hide the lack of hair.This advantage is achieved with the container object of this invention, due to the compact size of the diameter of the fixed quantity regulating filter and the regulating quantity filter of approximately 1,5 centimeters, unlike the filters of the existing containers in the market whose diameter is 3 centimeters.
- The application system functions directly from the flexible dispenser itself in order to apply hair fiber or hair concealer; it is applied upside down and passes through the openings present in the filters, which are left open according to the selected quantity level, without the hair fiber be pushed through any other air-pump device or intermediate reservoir, bulb or air bag or any of the systems mentioned in the previous paragraphs, nor are external tools used; these include, nozzles, tubes or channels; this completely prevents the hair fiber from becoming blocked; hair fiber, due to its nature, becomes thick, lumpy and forms obstructions in these devices; this gives the flexible dispenser the advantage of avoiding the problems associated with the incorrect application of the hair fiber and the need, should it arise, to eliminate it; It also prevents it from being visible and guarantees that the hair fiber is deposited in each hair, invisible and imperceptible with a perfect result.
- The container object of this invention is recyclable and respects the environment and does not use gas or batteries.
- The flexible dispenser for the application of hair fiber or hair concealer, by applying the fiber precisely and exactly on the place or area to treat and avoiding exterior tools from becoming blocked due to application, reduces the amount of fiber applied during each application; this means substantial savings with regards product application and to product use.
- The container object of this invention is lightweight, even comes in pocket size, and is quick and easy to use; this means you save time and avoid the application ritual of other hair-fiber devices; this makes it possible for the individual to apply hair fiber daily in order to conceal hair loss.
- Thanks to the flexible dispenser for the application of hair fiber or hair concealer the selection of levels according to the area to be treated is done in seconds, without the need to change channels, tubes and nozzles, which get dirty and make inconvenients. The application of hair fiber is precise and exact.
- Due to the application system, whose design is based on what previous studies and the use of filters which are approximately 1.5 centimeters in diameter, the holes located on the quantity filters, their combination with respect to the position of some holes with others in both filters when selecting the amount, as well as the size of the container and the fact that it is oval and elongated, with only its filters being cylindrical, the hair fiber comes out of the hair-fiber dispenser, object of the invention, without being thickened, without lumps, and totally fine; this achieves the desired result of its application being invisible to others.

The flexible dispenser for the application of hair fibers or hair concealer object of the invention includes a lower part called flexible dispenser, a fixed quantity regulating filter, an upper quantity regulating filter, fastener slots, various holes located in both filters and three selection levels for choosing the amount of fiber to apply. Below is a detailed description of these elements.
- One part called the flexible dispenser, located in the lower part of the container, where the hair fiber is stored; this flexible dispenser is connected to the fixed quantity regulating filter, via slots.
- A fixed quantity regulating filter, not movable, which is joined to the upper part of the flexible dispenser, and which makes up approximately half of the upper part of the container object of invention. This fixed regulating filter joined with the part called flexible dispenser has an elongated oval shape, and is not cylindrical; the part called flexible dispenser being greater in terms in aperture and diameter than the upper part of the fixed quantity regulating filter and the upper quantity regulating filter; nonetheless, its termination in the upper part is cylindrical, with a diameter of 1.5 centimeters, 8 holes and several slots are located in the upper part, which serve to hold and connect it to the upper quantity regulating filter, which is inserted into the upper part of the fixed quantity regulating filter; through this one can select the amount of hair fiber to apply.
- A movable upper quantity regulating filter, which is cylindrical and is inserted into the upper part of the fixed quantity regulating filter. The diameter of the upper quantity regulating filter is approximately 1.5 centimeters and has 12 holes located in the upper part.
- There are three quantity levels to choose from, level 1, level 2, and level 3; these are configured to very precisely, exactly, and cleanly dispense the hair fiber onto each of the hairs; this system prevents the application of hair fiber in unwanted areas (forehead, face, etc.), and thereby achieves the desired result of completely concealing hair loss while making the hair fiber unnoticeable.

Several holes are located in the each of the aforementioned filters; these, upon selecting the different application levels, produce a combination of openings or holes; their location have been studied according to the amount of hair fiber one wishes to apply and the compositional nature of the hair fiber so that the comes out fine, without clumps, and therefore can be applied so that it is invisible to others.

The amount of fiber to apply, is selected by turning the upper quantity regulating filter on the upper part of the fixed quantity regulating filter, creating a specific combination of openings of both filters for each of the of the three quantity levels selected; this allows the hair fiber to come out in different proportions, depending on the level selected; by being turned, the two filters produce a combination of between 4 and 8 openings or holes.

The individual, depending on the area to treat and the existing hair loss, chooses the amount of hair fiber to apply, level 1, level 2, or level 3, and positions the flexible dispenser upside down to apply the hair fiber or hair concealer on each of the hairs or areas with hair loss; the hair fiber is deposited on the upper part of the fixed quantity regulating filter and on the upper quantity regulating filter; the part called the flexible dispenser, located in the lower part of the device, is then lightly squeezed in order to apply the hair fiber or hair concealer. The hair fiber is directed towards the fixed quantity regulating filter and towards the upper quantity regulating filter by its own weight and nature, and directly comes out of the openings of the open holes according to the combination of the plurality of holes present in both regulating filters, their position and aperture depending on the amount of hair fiber to be applied.

The hair fiber is filtered upon penetrating the openings left open according to the quantity selected; the combinations are created by the 8 holes located in the upper part of the fixed regulating filter and the 12 holes in the upper part of the regulating filter; these combinations differ depending on the amount of hair fiber to be applied. Once filtered, the hair fiber comes out loosely and without lumps; it does not get stuck or thickened in any conduit, nozzle, channel, or any other tool located in the container since the container itself lacks these. By adhering to the hairs the user wishes, it solves the problem of hair loss in a way which is unnoticeable by others.

The hair fiber is deposited or applied only on the area or hairs which the individual wishes it to be applied to, and not on any other area, with the amount of hair fiber applied being strictly controlled; this results in what the user desires when using the product, which is to conceal hair loss by applying hair fiber only on hairs or areas which really need it, without it falling on the forehead, other parts of the head, or skin in general.

The flexible dispenser for the application of hair fiber or hair concealer, object of the invention, is made of a flexible material so that it be correctly applied with just slight pressure; it is oval and elongated in shape, until the upper part of the fixed quantity regulating filter; there it connects with the quantity regulating filter; both filters are cylindrical in shape at this point; the compact size of the diameter of the filters of approximately 1.5 centimeters, located in the upper part of the container object of invention, allows for complete precision when applying hair fiber to the area desired.

For the purposes of this invention, the flexible dispenser for the application of hair fiber or hair concealer conceals hair loss by applying the exact amount of hair fiber desired and with perfect precision on the area to be treated; this prevents others from noticing its use, which means that its use remains a secret; this satisfies what someone purchasing this product wants, which is to conceal hair loss without people knowing that a product has been used.
The technique experts should appreciate that the concept of this description that the terminology employed in this document is for descriptive ends and should not be considered to be all limiting.

### BRIEF DESCRIPTION OF THE DRAWINGS

To complement the description being made and with the aim of helping one to better understand the invention's characteristics, there is a series of drawings included in said description which show the following:

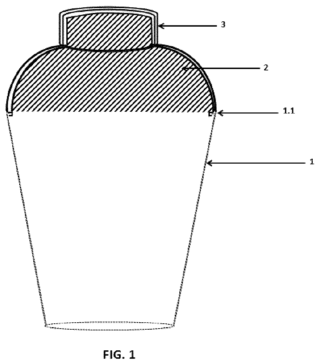

Figure 1. - Shows a complete frontal view of the flexible dispenser for the application of hair fiber or hair concealer, comprised of: the flexible dispenser (1); the tab (1.1) which connects the fixed quantity regulating filter (2) with the part which is the object of the invention, the flexible dispenser (1); the fixed quantity regulating filter (2) and the upper quantity regulating filter (3).

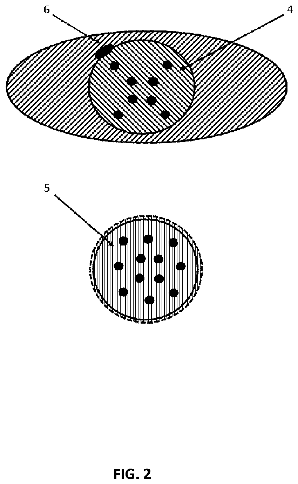

Figure 2. - Shows a top view of the location of the 8 holes (4) located strategically in the fixed quantity regulating filter (2) and the location of the 12 holes (5) located strategically in the upper quantity regulating filter (3). At the same time it shows the selector slot for choosing quantities (6) located in the upper fixed quantity regulating filter (2) which connects to the upper quantity regulating filter (3) so that the individual can select each of the different quantity levels to apply.

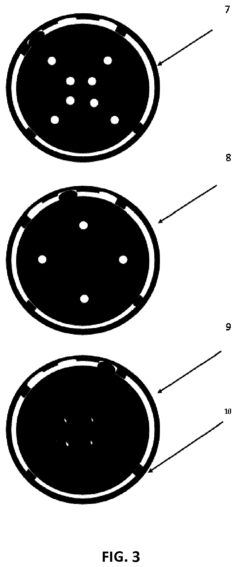

Figure 3.- Shows the quantities to select or the exit of the hair fiber or hair concealer according to the existing openings or holes with the combination of the holes produced by turning the upper quantity regulating filter (3) on the fixed quantity regulating filter (2) these being: Level 1 (7), level 2 (8) and level 3 (9). It also shows the slots (10) with which the upper quantity regulating filter is fixed (3) to the fixed quantity regulating filter (2) and the fitting slots(6) used to choose the amount of hair fiber or hair concealer to apply, as described in figure 2.

It also shows a number of open openings or holes and the aperture of the same, which vary between 4 and 8 open holes, for each of the levels, once the different quantity levels for applying hair fiber have been selected; These are level 1(7), level 2(8), and level 3(9); their use depends on which areas are to be treated and the individual's hair loss, keeping in mind the combination of the 12 holes (5) located in the upper quantity regulating filter (3) with the 8 holes(4) located in the fixed quantity regulating filter (2) caused by turning the upper quantity regulating filter (3) on the fixed quantity regulating filter (2).

### PREFERRED USE OF THE INVENTION

Figures 1, 2, and 3 show a section of a preferred use of the invention.

In figure 1, the flexible dispenser for the application of hair fiber or hair concealer stands out, with all the indispensable component parts for the preferred use of the invention being the following:
The part of the flexible dispenser for the application of hair fiber or hair concealer, called flexible dispenser, (1) is the part object of the invention which contains the hair fiber, and is connected via a tab (1.1) in its upper part, to the lower part of the fixed quantity regulating filter(2).

The lower part of the flexible dispenser for the application of hair fiber or hair concealer, called flexible dispenser (1) for the preferred use of the invention, is made of a flexible material which allows you to lightly squeeze it; furthermore, it has an oval and elongated, not cylindrical, section; it is in the upper part of the fixed quantity regulating filter (2) where the object of invention ends in a cylindrical fashion, i.e. in its filters; these have a diameter of approximately 1.5 centimeters, although other sections of the same, for example, with geometrical shapes may be object of this invention.

The upper quantity regulating filter (3) in the preferred use of the invention is inserted into the upper part of the fixed quantity regulating filter (2) the upper quantity regulating filter being (3) a mobile filter which turns on the fixed quantity regulating filter (2) in order to select the different amounts of hair fiber to apply, helped by the quantity-selection slot (6), described in figure 2 and the tabs (10) described in figure 3.

For the preferred use of the invention, the individual who wishes to apply hair fiber to conceal bald spots selects the amount of hair fiber to apply; the levels are Level 1 (7), level 2(8), and level 3 (9), as described in figure 3; the level selected depends on their needs or hair loss in the area they wish to treat, according to the area or need or hair loss.

Then the flexible dispenser for the application of hair fiber or hair concealer is placed face down, the flexible dispenser (1) containing the hair fiber is lightly squeezed, and said fiber comes out of the fixed quantity regulating filter holes (2). The hair fiber goes through the resulting holes or opening resulting from the combination of holes, located in the fixed quantity regulating filter (2) and the upper quantity regulating filter (3).

This is achieved by selecting the amount of hair fiber to apply, which produces the combination of the 8 holes (4), located in the fixed quantity regulating filter,(2) with the 12 holes (5),located in the upper quantity regulating filter, (3) according to the amount selected, which depends on the hair loss or area to treat; this is done by turning the upper quantity regulating filter (3) on the upper part of the fixed quantity regulating filter(2).

The final aperture of the openings or holes in the filters, depends on the amount of hair fiber to apply, as selected by the individual; this is based on the amount of hair loss they wish to conceal, so that the hair fiber comes fine, without being thickened, and without clumps after passing through the combination of holes in each of the filters; it also means that the exact amount needed for the area or hair which the individual wishes to treat comes out. These openings are open, semi-open, or closed, depending on the combination of holes located in the fixed quantity regulating filter (2) and the upper quantity regulating filter (3); these combinations are determined by selecting the amount of hair fiber which one wishes to apply.

In figure 2 one can see as an indispensable part of the preferred use of the invention, the top view of the fixed regulating filter (2) and the location of the 8 holes (4) located in the fixed quantity regulating filter (2) as well as the quantity-selector slot (6) which helps to connect the upper quantity regulating filter (3) with the upper part of the fixed quantity regulating filter (2); this allows the individual to choose one of the different amounts to apply by turning the upper quantity filter (3) on the upper part of the fixed quantity regulating filter(2) and via fastening slots in the upper quantity filter (10) shown in figure 3.

The fixed quantity regulating filter (2) of the preferred use of the invention, has 8 strategically-placed holes (4). The upper quantity regulating filter (3) of the preferred use of the invention has 12 strategically-placed holes (5).
The upper quantity regulating filter (3) is inserted into the fixed quantity regulating filter (2) through fastening slots in the upper quantity regulating filter (10), as shown in figure 3.

Once the upper quantity regulating filter (3) has been inserted into the fixed quantity regulating filter (2), turning the upper quantity regulating filter (3) on the fixed quantity regulating filter (2), allows one to select the different quantities of hair fiber or hair concealer to apply; this is done via the quantity-level selector slot (6). The opening of the 8 holes (4) and 12 holes (5) of both filters combine, depending on the different amounts of hair fiber to apply; these levels are Leve 1 (7), level 2 (8) and level 3 (9), and are shown in figure 3.

The exact amount of hair fiber or hair concealer is precisely applied (only on the area to treat) via the strategic combination of the holes of both filters, in other words via the combination of the 8 holes in the fixed quantity regulating filter (4) and the location of the 12 holes in the upper quantity regulating filter (5), and keeping in mind the small size of the diameter of the filters of approximately 1.5 centimeters, and the light pressure applied on the flexible dispenser (1).

As can be seen in figure 2, each filter has the necessary holes; these are strategically placed so that once super-imposed, they regulate the amount of hair fiber or hair concealer which is applied to existing hair loss or the area to treat in a precise manner.

Figure 3 shows as an essential part of preferred use the combination resulting from the super-imposition of the 8 holes (4) located in the fixed quantity regulating filter (2) and the 12 holes (5) located in the upper quantity regulating filter (2) in the three positions corresponding to the amount to be applied. These quantity levels of hair fiber to apply are level 1 (7), level 2 (8) and level 3 (9).

This figure of the preferred use of the invention describes the position of the openings or holes according the quantity selected, the position and aperture of between 4 and 8 holes resulting from the combination of holes, as well as where the hair fiber or hair concealer will come out to be applied, once every one of the hair-fiber application levels have been selected and after the user has lightly squeezed the flexible dispenser (1) with their fingers according to the amount of hair fiber which needs to be applied; Level 1 (7) results in 8 openings or holes remaining completely open, level 2 (8) in 4 openings or holes remaining completely open and level 3 (9) in 4 holes remaining approximately a quarter open, as seen in figure 3.

Variations in material, shape, or disposition of the component elements, described in a non-encompassing manner do not alter the essential design of the invention, basing this to proceed to its production by an expert.

The flexibility of the dispenser material, the small diameter of the filters of approximately 1.5 centimeters, the amount of holes in each filter, their combination upon selecting the level of hair fiber to apply and the position and opening of the holes, which are strategically placed to work in combination with one another bear mentioning; this is essential so that once the amount has been selected, the exact aperture for expelling hair fiber is achieved through the regulating quantity filters without needing for it to go through any intermediate container, conduct, nozzle, or channel which may cause the hair fiber to get stuck upon expulsion; this way the application of the hair fiber on the hairs is done precisely and exactly, without weighing the hair down and without clumping; it also causes the hair fiber to only be deposited exactly where the individual wants it to be, achieving a result which is completely unnoticeable, thereby satisfying the needs of the individual who wishes to keep its use a secret.

## Claims

1. The flexible dispenser to applying fiber or hair concealer, **characterized by** its flexible material and including, at least; a flexible dispenser (1); a fixed-quantity regulating filter (2); and a upper regulating quantity filter (3) which is located on top of the fixed-quantity regulating filter (2).
It is also **characterized by** the diameter of the upper part of the fixed-quantity regulating filter (2) and the upper regulating quantity filter (3) being approximately 1.5 centimeters and cylindrical in shape. It is also **characterized by** the upper part of the fixed-quantity regulating filter (2) having 8 holes (4) strategically located. At the same time, the upper quantity regulator filter (3) has 12 holes (5), also strategically located. The upper quantity regulator filter (3) is meant to be used to select, upon being turned on the fixed-quantity filter (2), the three quantity levels or output levels for existing fiber or hair concealer via the quantity-level selector slot (6). The quantity-selection levels are level 1º (7), level 2º (8) and level 3º (9) are distinguished from one another by their position, aperture and number of holes open in both filters upon being combined, an interval of between 4 and 8 holes depending on the selected quantity level.

2. The flexible dispenser for the application of hair fiber or hair concealer, according to claim 1, is **characterized by** the fixed quantity filter (2) being inserted via a tab (1.1) which connects the fixed quantity regulating filter (2) with the flexible dispenser (1) by pressure.

3. The flexible dispenser for the application of hair fiber or hair concealer, according to claim 1, is **characterized by** the upper quantity regulating filter (3) being inserted into the fixed quantity regulating filter (2) via slots (10) which attach the upper quantity regulating filter (3) to the fixed quantity regulating filter (2) and slots (6) and (10) (6) which fasten the filter and select the amount level.

4. The flexible dispenser for the application of hair fiber or hair concealer, according to claim 1, is **characterized by** the upper quantity regulating filter(3) and the fixed quantity regulating filter (2) containing holes (4) located strategically in the fixed quantity regulating filter (2) and in the upper quantity regulating filter (3); these determine the amount of hair fiber or hair concealer released, according to the level selected; the levels are level 1º (7), level 2º (8) and level 3º (9).

5. The flexible dispenser for the application of hair fiber or hair concealer, according to claim 1, is **characterized by** being able to select the different levels of hair fiber or hair concealer to apply (level 1º (7), level 2º (8) and level 3º (9) once the upper quantity regulating filter (3) is inserted into the fixed quantity regulating filter by turning the upper quantity regulating filter (3) on the fixed quantity regulating filter (2), and via the quantity selector slots(6) and the fastening slots (10) in the internal part of the upper quantity regulating filter (3) and in the outer part of the fixed quantity regulating filter (2), there existing said quantity levels in the internal part of the the upper quantity regulating filter (3) and in the outer part of the fixed quantity regulating filter (2).

6. The flexible dispenser (1) for the application of hair fiber or hair concealer, according to claim 1, is **characterized by** the turning of the upper quantity regulating filter (3) on the fixed quantity regulating filter (2) resulting in the holes of both filters superimposing on each other and combining with three different aperture levels ((level 1º (7), level 2º (8) and level 3º (9)), according to the levels selected with the slot for selecting quantity levels (6), resulting in each of the levels expelling a different amount of hair fiber or hair concealer to be applied, depending on the amount needed and with exactitude and precision on the area to treat.

7. The flexible dispenser for the application of hair fiber or hair concealer, according to claim 1, is **characterized by** the fact that once the individual selects the amount of hair fiber or hair concealer to apply according to the three existing levels by turning the upper quantity regulating filter (3) on the fixed quantity regulating filter (2), using the quantity-selector slot (6), and applying pressure to the flexible dispenser (1) with their hands, the hair fiber or hair concealer comes out via the gaps produced by the holes in the fixed quantity regulating filter (4) and the holes in the upper quantity regulating filter (5) left open by being combined; the hair fiber or hair concealer is then applied in a precise manner, and exactly where desired.
